# EUROPEAN PATENT SPECIFICATION

(11) **EP 3 422 938 B1**
(45) Date of publication and mention of the grant of the patent: **22.04.2020**
(21) Application number: 17710152.4
(22) Date of filing: 02.03.2017
(51) Int. Cl.: G16H 10/00

(54) **PERSONALIZED FOOD FOR DYSPHAGIA MANAGEMENT**
PERSONALISIERTE NAHRUNG FÜR DYSPHAGIA MANAGEMENT
ALIMENTATION PERSONNALISÉE POUR LA GESTION DE LA DYSPHAGIE

(30) Priority: 03.03.2016 EP 16158553
(43) Date of publication of application: 09.01.2019
(73) Proprietor: Société des Produits Nestlé S.A., 1800 Vevey (CH)
(72) Inventor: LE REVEREND, Benjamin, 74500 Neuvecelle (FR); BURBIDGE, Adam, 1273 Arzier (CH); RAMAIOLI, Marco, Lonon SW11 2LZ (GB); ENGMANN, Jan, 1012 Lausanne (CH)
(74) Representative: Chautard, Cécile
(86) International application number: PCT/EP2017/054850
(87) International publication number: WO 2017/149056

(56) References cited:
- US-A1- 2010 209 893
- ALI S ET AL: "In-Vivo Analysis of Aroma Release while Eating Food: A Novel Set-up for Monitoring On-line Nosespace Air", INTERNATIONAL CONFERENCE ON PROTON TRANSFER REACTION MASSSPECTROMETRY AND ITS APPLICATIONS, XX, XX, 18 January 2003 (2003-01-18), pages 161-164, XP002345928,
- DD ROBERTS ET AL: "Comparison of nosespace, headspace, and sensory intensity ratings for the evaluation of flavor absorption by fat.", J AGRIC FOOD CHEM., 3 May 2003 (2003-05-03), pages 3636-3642, XP055168043, DOI: 10.1021/jf026230+

## Description

### FIELD OF THE INVENTION

The present invention relates to the field of swallowing difficulties. In particular, the invention relates to methods for selecting a suitable food product to administer to a subject having dysphagia.

### BACKGROUND OF THE INVENTION

Swallowing is a basic physiological function which is necessary for survival. Disorders in which subjects have difficulty swallowing are often associated with high mortality rates, due in part to starvation or dehydration. Failure to swallow properly may also lead to aspiration of food particles into the lungs, which often leads to pneumonia. Swallowing disorders may be referred to as dysphagia.

Conditions leading to dysphagia include, for example, oral cancer, stroke (cerebral infarction and haemorrhage) and craniocerebral trauma. A high proportion of such subjects develop dysphagia and subsequently aspiration pneumonia.

Modified or thickened fluids are often recommended as a dysphagia management strategy by dieticians and speech and language therapists. However, the use of thickened fluids in the management of dysphagia is complex and different levels of thickness are suited and preferred for different patients. Determining the most appropriate consistency of food product for an individual patient is often achieved by subjective feedback and consultation between healthcare professionals and patients.

It would be advantageous to provide an objective method for determining the most appropriate food product to administer to a subject with dysphagia.

A number of methods are currently in clinical use in order to monitor swallowing in a subject. In X-ray video fluoroscopy, the subject swallows a food product containing a contrast medium. The swallowing process is then recorded as a video using fluoroscopy. For analysis, the video may be studied in slow motion.

Typically such a method may involve obtaining X-ray images over a period of 4-5 seconds at a rate of 15 images per second. This involves exposure to a significant dose of X-rays, with associated risks. The procedure is also technically complex and is not quantitative.

An alternative method is endoscopy, sometimes referred to as fibre optic endoscopic examination of swallowing (FEES). By introducing a flexible endoscope through the nose of the subject, the ingestion of various food types can be studied. In some cases a fluorescent dye may be included in a fluid which is then swallowed, in order to facilitate visualization of residues in the oropharyngeal cavity. A disadvantage of endoscopy is that images cannot be obtained throughout the entire ingestion process, because the tongue and posterior pharyngeal wall tend to obscure the view during swallowing itself. Another method for monitoring swallowing in real time is ultrasound sonography. However, this method is of limited use, in particular due to difficulties in detecting particular tissue structures and food residues.

Accordingly, there is a need for new methods for monitoring swallowing, for instance in order to select the most appropriate food product to administer to a subject with dysphagia.

Document US2010209893 discloses a process of selecting food based on an intrinsic characteristic of the food product, such as viscosity, for people suffering from dysphagia.

### SUMMARY OF THE INVENTION

The aim of the present invention is achieved by subject-matter specified in the independent claims. Particular embodiments of the invention are specified in the dependent claims.

Accordingly, in a first aspect of the present invention there is provided a method for selecting a food product for a subject having dysphagia according to claim 1.
(a) administering one or more food products comprising a volatile compound to the subject;
(b) detecting release of the volatile compound in exhaled breath of the subject during and/or after swallowing of the one or more food products;
(c) selecting the food product based on the release of the volatile compound in exhaled breath of the subject.

The method may comprise comparing the release of the volatile compound to a control.

By way of example, the control may be the corresponding release of the volatile compound when the food product is administered to a subject that does not have any condition which causes difficulty swallowing (e.g. does not have dysphagia).

In the present method, the detection of a volatile compound may be determined for a number of different food products following and/or during swallowing. The detection profile for the volatile compound may then compared between the test subject and a control level for each given food product. The food product for which the detection profile is most similar between the test subject and the control level may then be selected.

The method may further comprise administering the selected food product to the subject.

In one embodiment, the food product comprising the volatile compound is spiked or sprayed with the volatile compound.

The food products may be selected from a liquid or semi-solid food product. The food product may have syrup or nectar consistency, custard or honey consistency, or pudding consistency. The food product may be selected from water, milk, soup, yogurt, juice, coffee, tea, soda, or combinations thereof. The volatile compound may be detected using mass spectrometry, a breath analyser / breathalyser or a microfluidics chip. The volatile compound may be detected by a method selected from a group consisting of proton transfer reaction mass spectrometry (PTR-MS, either PTR-QMS or PTR-TOF-MS), atmospheric-pressure chemical ionization mass spectrometry (APCI-MS), gas chromatography mass spectrometry (GC-MS) and gas chromatography ion-mobility mass spectrometry (GC-IMS).

In one embodiment, levels of the volatile compound in exhaled breath after swallowing are indicative of residues of the food product in the oropharyngeal cavity of the subject. In one embodiment, levels of the volatile compound in exhaled breath after swallowing are indicative of aspiration of the food product by the subject.

The volatile compound may be selected from a group consisting of ethanol, limonene and ethyl butyrate. In a particular embodiment the volatile compound is ethanol.

The method may further comprise monitoring phases of the swallowing process in the subject, for example by ultrasound imaging and/or ultrasound Doppler velocimetry.

### BRIEF DESCRIPTION OF THE FIGURES

Figure 1 - Typical results obtained from the coupling of aroma release measured using PTR-TOF-MS with the different phases of oral processing. The circle marks a 5mV trigger recorded using the analogue input.
Figure 2 - Profile of volatile compound detection using a high viscosity fluid (M65 - molasses in orange juice and spiked with limonene)
Figure 3 - Profile of volatile compound detection using a low viscosity fluid (water spiked with limonene)
Figure 4 - Signal decay in a range of food products

### DETAILED DESCRIPTION OF THE INVENTION

The present invention relates to a method for selecting a food product to administer to a subject having dysphagia. The method advantageously enables a health care practitioner, for example in a hospital, to select a suitable food product for a subject with dysphagia. Moreover, the method may be quantitative, allows analysis both during and after swallowing itself and does not involve the risks associated with methods involving X-rays.

Thus, the present invention provides a method for selecting a food product for a subject having dysphagia comprising:
(a) administering one or more food products comprising a volatile compound to the subject;
(b) detecting release of the volatile compound in exhaled breath of the subject during and/or after swallowing of the one or more food products;
(c) comparing the release of the volatile compound to a control
(d) repeating steps (a) to (c) for at least one further food product comprising a volatile compound; and
(e) selecting the food product for which the release of the volatile compound in exhaled breath of the subject is most similar to the control.

Preferably, following the selection of a food product using the present method, the food product is administered to the subject in a form which does not comprise the volatile compound. For example, if the volatile compound has been sprayed or spiked into the food product for use in steps (a) to (e) of the method, once the most suitable food product has been selected it is administered to the subject in a form which does not comprise the sprayed or spiked volatile compound.

The method may comprise comparing the release of a volatile compound between subjects with dysphagia, and control subjects (e.g. wherein the control subjects are healthy subjects that do not have dysphagia) for at least one, at least two, at least three, at least four, or at least five or more food products.

The method of the present invention preferably relates to selecting a food with an appropriate rheology (e.g., honey, nectar or pudding) for administration to a subject. By way of example, one or more foods comprising a volatile compound may be administered to a subject suffering from dysphagia. The release of the volatile compound in exhaled breath during and/or after swallowing of the food product may be compared to the corresponding release of the volatile compound in a healthy subject. The food selected may be the one wherein the swallowing profile as assessed by the released volatile compound is most similar between the subject suffering from dysphagia and the healthy subject.

### Monitoring swallowing

The method of selecting a food according to the present invention involves monitoring swallowing in a subject. By "monitoring swallowing" it is intended to include any method which involves studying the swallowing process. In particular, the method may be used to detect incomplete or partial swallowing (e.g. by detecting the presence of food residues in the oropharyngeal cavity) and/or aspiration.

The normal swallowing of a human (or mammal) involves three distinct phases which are interdependent and well-coordinated: (i) the oral, (ii) the pharyngeal, and (iii) the esophageal phases. In the oral phase, which is under voluntary control, food that has been chewed and mixed with saliva is formed into a bolus for delivery by voluntary tongue movements to the back of the mouth, into the pharynx. The pharyngeal phase is involuntary and is triggered by food/liquid bolus passing through the faucial pillars into the pharynx. Contraction of the three constrictors of the pharynx propels the bolus towards the upper esophageal sphincter. Simultaneously, the soft palate closes the nasopharynx. The larynx moves upwards to prevent food or liquid passing into the airway, which is aided by the backward tilt of the epiglottis and closure of the vocal folds. The esophageal phase is also involuntary and starts with the relaxation of the upper esophageal sphincter followed by peristalsis, which pushes the bolus down to the stomach.

The method of the present invention may involve monitoring the phases of the swallowing process in a subject. As used herein 'monitoring of the phases of the swallowing process' is synonymous with observing or visualising the phases of the swallowing process.

Monitoring of the phases of the swallowing process in a subject may be performed using any device or method which enables the phases of the swallowing process to be observed. For example, the swallowing process may be observed using magnetic resonance imaging (MRI), ultrasound imaging and/or ultrasound Doppler velocimetry techniques.

### Dysphagia

The method of the present invention allows a food product e.g. a food with an appropriate rheology to be selected for administration to a subject having a medical condition which causes difficulty in swallowing.

Dysphagia refers to the symptom of difficulty in swallowing. General causes of dysphagia have been identified and include, but are not limited to, a decreased ability to swallow, the tongue not exerting enough pressure on the soft palate, abnormal epiglottis behaviour, etc. The consequences of untreated or poorly managed oral pharyngeal dysphagia can be severe, including dehydration, malnutrition leading to dysfunctional immune response, and reduced functionality, airway obstruction with solid foods (choking), and airway aspiration of liquids and semi-solid foods, promoting aspiration pneumonia and/or pneumonitis.

Esophageal dysphagia affects a large number of individuals of all ages, but is generally treatable with medications and is considered a less serious form of dysphagia. Esophageal dysphagia is often a consequence of mucosal, mediastinal, or neuromuscular diseases. Mucosal (intrinsic) diseases narrow the lumen through inflammation, fibrosis, or neoplasia associated with various conditions (e.g. peptic stricture secondary to gastroesophageal reflux disease, esophageal rings and webs [e.g. sideropenic dysphagia or Plummer-Vinson syndrome], esophageal tumors, chemical injury [e.g., caustic ingestion, pill esophagitis, sclerotherapy for varices], radiation injury, infectious esophagitis, and eosinophilic esophagitis). Mediastinal (extrinsic) diseases obstruct the esophagus by direct invasion or through lymph node enlargement associated with various conditions (tumors [e.g., lung cancer, lymphoma], infections [e.g., tuberculosis, histoplasmosis], and cardiovascular [dilated auricula and vascular compression]). Neuromuscular diseases may affect the esophageal smooth muscle and its innervation, disrupting peristalsis or lower esophageal sphincter relaxation, or both, commonly associated with various conditions (achalasia [both idiopathic and associated with Chagas disease], scleroderma, other motility disorders, and a consequence of surgery [i.e., after fundoplication and antireflux interventions]). It is also common for individuals with intraluminal foreign bodies to experience acute esophageal dysphagia.

Oral pharyngeal dysphagia, on the other hand, is a very serious condition and is generally not treatable with medication. Oral pharyngeal dysphagia also affects individuals of all ages, but is more prevalent in older individuals. Worldwide, oral pharyngeal dysphagia affects approximately 22 million people over the age of 50. Oral pharyngeal dysphagia is often a consequence of an acute event, such as a stroke, brain injury, or surgery for oral or throat cancer. In addition, radiotherapy and chemotherapy may weaken the muscles and degrade the nerves associated with the physiology and nervous innervation of the swallow reflex. It is also common for individuals with progressive neuromuscular diseases, such as Parkinson's Disease, to experience increasing difficulty in swallowing initiation. Representative causes of oropharyngeal dysphagia include those associated neurological illnesses (brainstem tumors, head trauma, stroke, cerebral palsy, Guillain-Barre syndrome, Huntington's disease, multiple sclerosis, polio, post-polio syndrome, metabolic encephalopathies, amyotrophic lateral sclerosis, Parkinson's disease, dementia), infectious illnesses (diphtheria, botulism, Lyme disease, syphilis, mucositis [herpetic, cytomegalovirus, Candida, etc.]), autoimmune illnesses (lupus, scleroderma, Sjogren's syndrome), metabolic illnesses (amyloidosis, cushing's syndrome, thyrotoxicosis, Wilson's disease), myopathic illnesses (connective tissue disease, dermatomyositis, myasthenia gravis, myotonic dystrophy, oculopharyngeal dystrophy, polymyositis, sarcoidosis, paraneoplastic syndromes, inflammatory myopathy), iatrogenic illnesses (medication side effects [e.g., chemotherapy, neuroleptics, etc.], post surgical muscular or neurogenic, radiation therapy, corrosive [pill injury, intentional]), Tardive Dyskinesia [A chronic disorder of the nervous system characterized by involuntary jerky movements of the face, tongue, jaws, trunk, and limbs, usually developing as a late side effect of prolonged treatment with antipsychotic drugs], and structural illnesses (cricopharyngeal bar, Zenker's diverticulum, cervical webs, oropharyngeal tumors, osteophytes and skeletal abnormalities, congenital [cleft palate, diverticulae, pouches, etc.]).

The method of the present invention may be used to select an appropriate food for a subject who has any one or more of the conditions recited above.

Dysphagia is not generally diagnosed although the disease has major consequences on patient health and healthcare costs. Individuals with more severe dysphagia generally experience a sensation of impaired passage of food from the mouth to the stomach, occurring immediately after swallowing. Among community dwelling individuals, perceived symptoms may bring patients to see a doctor. Among institutionalized individuals, health care practitioners may observe symptoms or hear comments from the patient or his/her family member suggestive of swallowing impairment and recommend the patient be evaluated by a specialist. As the general awareness of swallowing impairments is low among front-line practitioners, dysphagia often goes undiagnosed and untreated. Yet, through referral to a swallowing specialist (e.g., speech language pathologist), a patient can be clinically evaluated and dysphagia diagnosis can be determined.

Severity of dysphagia may vary from: (i) minimal (perceived) difficulty in safely swallowing foods and liquids, (ii) an inability to swallow without significant risk for aspiration or choking, and (iii) a complete inability to swallow. Many people with swallowing impairment do not seek medical care when symptoms are mild or unrecognized. For example, "silent aspiration," a common condition among elderly, refers to the aspiration of the oropharyngeal contents during sleep. People may compensate for less-severe swallowing impairments by self-limiting the diet. The aging process itself, coupled with chronic diseases such as hypertension or osteoarthritis, predisposes elderly to (subclinical) dysphagia that may go undiagnosed and untreated until a clinical complication such as pneumonia, dehydration, malnutrition (and related complications) occurs. Yet, the differential diagnosis of 'aspiration pneumonia' is not necessarily indicated as a result of current care practices.

### Aspiration

The term "aspiration" refers to the drawing of a foreign substance into the respiratory tract. Particularly, as used herein, aspiration refers to the drawing of a food product into the respiratory tract during swallowing.

Aspiration can occur before, during, or after the swallow. Aspiration occurs before the swallow in the case of a delayed or absent initiation of the swallow. It may also be the result of poor tongue control, which allows food to trickle into the pharynx while the patient is still chewing. Aspiration occurs during the swallow when the vocal folds fail to adduct or the larynx fails to elevate. Aspiration can occur after the swallow in several different circumstances: the patient may pocket food in the oral cavity, food may get stuck in the pharyngeal recesses or due to reduced laryngeal elevation, food may remain on top of the larynx.

### Subject

The term "subject" as used herein is interchangeable with "patient" or "individual". The term subject may refer to any animal, mammal or human having a medical condition that can benefit from a method of monitoring swallowing and selecting a food product as provided by the present invention.

For example the subject has a condition associated with dysphagia.

### Food product

An example of selecting a suitable food product for a subject having dysphagia comprises:
(a) administering one or more food products comprising a volatile compound to the subject;
(b) detecting release of the volatile compound in exhaled breath of the subject during and/or after swallowing of the one or more food products;
(c) selecting the food product based on the release of the volatile compound in exhaled breath of the subject.

The food product contains an amount of a volatile compound detectable to enable monitoring of swallowing. Inclusion of a volatile compound preferably does not modify the textural properties of the food products which have been specifically tailored to aid safe swallowing.

The food product may have previously been spiked or sprayed with a volatile compound.

'Spiked' or 'sprayed' is used herein to describe the addition of a volatile compound to the food product. The volatile compound may be essentially absent from the food product prior to the addition via spiking or spraying.

Spiking describes that the volatile compound is added within/into the food product. Spraying describes that the volatile compound coats all/or part of the surface of the food product.

The food product may be solid food product (for example a processed meat, fish or vegetable with a soft texture); a semi-solid food product or liquid food product.

In certain embodiments the food product may be a thickened liquid or a puree of solid foods, both of which have been shown to be an effective means of preventing choking and aspiration during the eating process. Thickened liquids are designed to have three properties: (i) a more cohesive bolus that can be maintained throughout the action of swallowing, (ii) slower delivery to the throat, thereby compensating for the increased period in which the swallowing reflexes prepare for the thickened liquid, and (iii) provide greater density to increase awareness of the presence of food or liquid bolus in the mouth.

Thickened food products for dysphagia are often defined according to their consistency.

For example, the food product may have a syrup or nectar consistency, a custard or honey consistency or a pudding consistency.

Examples of classification systems used for product thickness for dysphagia management are provided by "The Australian fluid viscosity scale" (Nutrition & Dietetics; Special Issue: Australian Standardised Terminology and Definitions for Texture Modified Foods and Fluids Volume 64, Issue Supplement s2, pages S53-S76, May 2007); "The USA fluid viscosity scale" (Matta et al.; J Am Diet Assoc 2006; 106: 1049-54) and "The UK fluid viscosity scale" (British Dietetic Association. National Descriptors for Texture Modification in Adults. Joint working party of The British Dietetic Association and the Royal College of Speech and Language Therapists, May 2002.)

The food product may be, for example, water, milk, soup, yogurt, juice, coffee, tea, soda, or combinations thereof.

In some embodiments, the food product may comprise starch or gum thickeners (thickening product). For example, the food product may be a beverage or liquid food which comprises a starch or gum thickener. The presence of starch or gum thickeners increases the viscosity of the beverage or liquid food and thus aids swallowing.

In certain embodiments, the volatile compound may be provided in the thickening product.

Examples of thickening products which may be used to thicken a food product are described in WO 2013/160207, WO 2013/087916 and WO 2013/087918.

Briefly, WO 2013/160207 describes a thickened composition having a xanthan gum thickening component, and orally administering the composition to an individual having a swallowing impairment. It is described that the administration of a thickened composition including a xanthan gum thickening component increases the efficacy of a swallow response by decreasing the presence of pharyngeal residue while at least maintaining swallowing safety. The xanthan gum is food grade and can be commercially obtained from numerous suppliers. Xanthan gum is a high molecular weight, long chain polysaccharide composed of the sugars glucose, mannose, and glucuronic acid. The backbone is similar to cellulose, with added side chains of trisaccharides. The compositions contain xanthan gum in an amount ranging from about 0.5 g to about 8 g, about 1 g to about 7 g, about 2 g to about 6 g, or about 3 g to about 4 g, per every 100 mL of a liquid carrier (e.g., water). In an embodiment, the compositions contain xanthan gum in an amount ranging from about 1.2 g to about 6 g. Thickening compositions comprising xanthan gum are available commercially, for example NestleHealthScience Resource® ThickenUPClear™.

Thus, in some embodiments the food product comprises a thickening composition having a xanthan gum thickening component. The food product may consist of a thickening composition having a xanthan gum thickening component. The xanthan gum thickening component may comprise the volatile compound.

The food product may comprise or consist of a ThickenUPClear™ food product.

WO 2013/087916 describes nutritional products having improved cohesiveness of food boluses. The nutritional products may include nutritional compositions and high molecular weight, water-soluble polymers such that the nutritional products have extensional viscosities that provide improved cohesiveness to the nutritional products and Trouton ratios of at least 6. The method for making such a nutritional composition comprises providing a nutritional composition and adding a food grade polymer to the nutritional composition to form a nutritional product having a Trouton ratio that is at least 6. The food grade polymer may be selected from plant extracted gums, plant-derived mucilages and combinations thereof. The plant extracted gums may furthermore be selected from okra gum, konjac mannan, tara gum, locust bean gum, guar gum, fenugreek gum, tamarind gum, cassia gum, acacia gum, gum ghatti, pectins, cellulosics, tragacanth gum, karaya gum, or any combinations thereof. Further, the plant-derived mucilages may be selected from the group consisting of kiwi fruit mucilage, cactus mucilage (Ficus indica), psyllium mucilage (Plantago ovata), mallow mucilage (Malva sylvestris), flax seed mucilage (Linum usitatissimum), marshmallow mucilage (Althaea officinalis), ribwort mucilage (Plantago lanceolata), mullein mucilage (Verbascum), cetraria mucilage (Lichen islandicus), or any combinations thereof.

Thus in certain embodiments of the present invention, the food product may comprise water-soluble polymers such that the food product has improved cohesiveness and a Trouton ratio of at least 6.

### Volatile Compound

The volatile compound may be any volatile compound which can be detected using the present method. The volatile compound may be any compound which can be detected in the exhaled breath of a subject during and/or after swallowing.

The "volatile compound" may be a compound with a high vapour pressure at room temperature. The volatile compound can be released from a matrix and can be found in the headspace surrounding the product. The high vapour pressure results from a low boiling point and/or a low solubility in the matrix, which causes large numbers of molecules to evaporate or sublimate from the liquid or solid form of the compound and enter the surrounding air. Molecules which enter the surrounding air may be referred to as the gas fraction.

Preferably, the volatile compound is non-toxic at the levels at which it is administered to the subject.

Preferably, the volatile compound is present in the food product at a concentration which yields gas fractions in the part per trillion (ppt), parts per billion (ppb), parts per million (ppm) range or parts per thousand (‰).

The food product may comprise at least one, at least two, at least three, at least four or at least five or more volatile compound as defined herein. The food product may comprise one, two, three, four, five or more volatile compounds.

In certain embodiments, the volatile compound is selected from a group consisting of ethanol, limonene and ethyl butyrate.

The volatile compounds may be incorporated into a food product as flavour compounds. The volatile compound may occur naturally in fruits (e.g. limonene in orange fruit). Volatile compounds cover a range of volatility and lipophilicity. Table 1 below shows the volatility and lipophilicity of exemplified volatile compounds (database Episuite 4.1).

| Compounds | CAS | Lipophilicity: Oil / Water partition coefficient log Kow [w/v] | Volatility: Air/water partition coefficient log K [w/v] |
|---|---|---|---|
| Ethanol | 64-17-5 | -0.31 | -3.69 |
| Vanillin | 121-33-5 | 1.05 | -8.47 |
| 2-Methylbutanal | 96-17-3 | 1.23 | -2.20 |
| 3-Methylbutanal | 590-86-3 | 1.23 | -2.19 |
| 2-Methylpropanal | 78-84-2 | 0.74 | -2.31 |
| 2,3-Pentanedione | 600-14-6 | -0.85 | -4.97 |
| Isoamylacetate | 123-92-2 | 2.26 | -1.65 |
| 2,3-Butanedione | 432-03-8 | -1.34 | -5.09 |
| Acetaldehyde | 75-07-0 | -0.17 | -2.56 |
| Ethylbutyrate | 105-54-4 | 1.85 | -1.79 |
| Methanethiol | 74-93-1 | 0.78 | -0.97 |
| Dimethylsulfide | 75-18-3 | 0.92 | -1.49 |
| Limonene | 138-86-3 | 4.38 | 0.12 |

In one embodiment, the volatile compound may have an air/water partition coefficient (log K [w/v]) of -10 to +1. For example the volatile compound may have an air/water partition coefficient (log K [w/v]) of -5 to +1.

In one embodiment, the volatile compound may have an oil/water partition coefficient (log Kow [w/v]) of -2 to +5.

Preferably, the volatile compound is ethanol.

In certain embodiments the concentration of ethanol in the food product is similar to that which occurs naturally in orange juice. For example, the concentration of ethanol may be, for example 0.05% to 0.5%. Preferably, the concentration of ethanol is 0.1%.

The food product may comprise a polar and a non-polar volatile compound.

The term 'polar' is used herein according to its conventional meaning to refer to a molecule having an electric dipole or multipole moment.

Polar compounds are hydrophilic and highly soluble in water e.g. ethanol. Non-polar compounds are lipophilic and have low solubility in water e.g. limonene.

The volatile compound may be spiked into or sprayed onto the food product.

In certain embodiments, the present invention provides the advantage that only low levels of the volatile compound in the food product are required, compared for example to the level of a fluorescent dye may be included in a fluid to facilitate FEES.

Preferably, the volatile compound is spiked into or sprayed onto the food product at a known level/amount/concentration such that the level of release of the volatile compound in exhaled breath following swallowing of the food product can be compared between different tests. For example the level of release may be compared between a test and a control subject or between tests performed on the same subject on different occasions (see below).

### Detecting Release

The present method involves detecting the release of a volatile compound in exhaled breath during and/or after swallowing of a food product.

The detection may be performed using any method or apparatus which is suitable for determining the level of the volatile compound present in exhaled breath (e.g. any method or apparatus that can determine if the volatile compound is present in exhaled breath and/or if the level of volatile compound is decreasing in subsequent exhalations and/or the rate at which the level of volatile compound detected in exhaled breath decreases).

Methods and apparatus which are suitable for detecting the release of the volatile compound are well-known in the art. For example, in some embodiments the volatile compound may be detected using mass spectrometry, a breathalyser or a microfluidics chip.

In certain embodiments the release of the volatile compound is detected by mass spectrometry. For example, the release may be detected by a method selected from proton transfer reaction mass spectrometry (PTR-MS), atmospheric-pressure chemical ionization mass spectrometry (APCI-MS), and gas chromatography ion-mobility mass spectrometry (GC-IMS).

The volatile compound may be detected on-line in real time by a time resolved method selected from, for example, PTR-MS, APCI-MS, and GC-IMS.

In certain embodiments, the release of the volatile compound is detected by PTR-MS.

In certain embodiments, the release of the volatile compound is detected by GC-IMS.

The volatile compound may be detected by 'nosespace' analysis, which refers to the detection of volatile compound in breath exhaled from the nose. The volatile compound may be detected by 'mouthspace' analysis, which refers to the detection of volatile compound in breath exhaled from the mouth. Breathalysers / breath analysers which detect volatile compounds in the exhaled breath of a subject are well known in the art, for example to detect ethanol. Such breathalysers are described, for example, in EP1584924, US 4770026 and WO 2010/009406. The skilled person will appreciate that such devices are suitable for use in a method according to the present invention. In addition, such breathalysers can be readily modified in order to detect alternative volatile compounds which may be detected in the method according to the present invention. Microfluidics devices for assaying components of exhaled breath are also known in the art (see Li et al; Anal Chem. 2012 Feb 7;84(3):1288-93; Fu et al; Cancer Med. 2014 Feb;3(1):174-81 and https://www.lcaos.eu)

The detection of a volatile compound in exhaled breath after swallowing may be indicative of residues of the food product in the oropharyngeal cavity and/or indicative of aspiration of the food product by the subject. Such detection is indicative that the subject has problems swallowing the food product. In certain embodiments the level of volatile compound detected in the exhaled breath of a subject during and/or after swallowing may be compared to a control level.

Reference to a "control" broadly includes data that the skilled person would use to facilitate the accurate interpretation of technical data. As such, "control level" is interchangable with "reference level".

The level or levels of volatile compound in the exhaled breath of a subject may be compared to the respective level or levels of the same volatile compound in one or more cohorts (populations/groups) of control subjects selected from a subject cohort wherein the subjects have been diagnosed with a condition which causes difficulty in swallow (e.g. dysphagia) and a subject cohort wherein the subjects have been predetermined not to have any condition which causes difficulty swallowing (e.g dysphagia). In a preferred embodiment, the cohort of control subjects is a cohort in which the subjects have been predetermined not to have any condition which causes difficulty swallowing (e.g dysphagia).

The control level may represent the level of volatile compound detected in the exhaled breath of a control cohort, wherein the same food product and same volatile compound are administered to the test subject and the control subjects. As the skilled person will recognise, the total amount and concentration of the food product and volatile compound (along with all other variables) should be kept as consistent as possible between the test subject and control subject(s).

In some embodiments, control or reference levels for the detection of a given concentration of a particular volatile compound, administered in a given food product, may be stored in a database and used in order to interpret the results of the method as performed on the subject.

Inefficient swallowing or dysphagia may be associated with;
i) increased levels of volatile compound detected in exhaled breath during and/or after swallow;
ii) levels of volatile compound which are detectable in an increased number of exhalations following swallow;
iii) levels of volatile compound which are detectable for a longer time period following swallow;
iv) a more gradual decrease in levels of volatile compound detectable in subsequent exhalations following swallow (e.g rate of depletion breath by breath) ; and/or
v) an increased time period between the initiation of swallowing and the detection of volatile compound in the first exhalation following swallow;
in comparison to reference/control levels in a subject/cohort with efficient swallowing.

Outputs, such as those described in (i) - (v) above, may be referred to as the detection profile for a volatile compound in a given food product.

The level of volatile compound may be quantified by amplitude or area under the curve methodologies or by calculating the level and/or ratio of the volatile compound compared to a reference compound.

In the present method, the detection of a volatile compound may be determined for a number of different food products following and/or during swallowing. The detection profile for the volatile compound is then compared between the test subject and a control level for each given food product. The food product for which the detection profile is most similar between the test subject and the control level is then selected.

By way of example, if the detection profile of the test subject and the control level differs to a significant extent (e.g. assessed using one or more of the features outlined in (i) - (v) above), this may indicate that the food product being tested is unsuitable for administration to that subject because it causes difficulties with swallowing. In contrast, if the detection profile between of the test subject and the control level is similar (e.g. assessed using one or more of the features outlined in (i) - (v) above) this may indicate that the food product being tested is suitable for administering to that subject because it is associated with less difficulty in swallowing. In some embodiments, it may be that all food products tested are associated with swallowing difficulties and the food product selected may be the one in which the smallest differences between the detection profile of the test subject and the control.

### Residues

The term "residues" refers to deposits of the food product which remain present in the subject's oropharyngeal cavity after swallowing or are aspirated into the subject's respiratory tract. Volatile compound which is present on/in the food product will be released from the deposits of food product which remain present in the oropharyngeal cavity, or are aspirated, and can be detected by the method of the present invention.

As a simple illustration, the more residues there are in the oropharyngeal cavity, the more volatile compound will be released, as a function of the residues surface area and volatile partitioning.

In certain embodiments, volatile compound released from aspirated residues are detected by the present method. The method of detection may be selected from proton transfer reaction mass spectrometry (PTR-MS), atmospheric-pressure chemical ionization mass spectrometry (APCI-MS), and gas chromatography ion-mobility mass spectrometry (GC-IMS).

### Examples

### Example 1 - Detection of volatile compounds in breath

### Products used

A commercial orange juice (Eckes-Granini Group GmbH, Nieder-Olm, Germany) was chosen due to the high content of volatile terpenes in orange juice and strong volatile signal obtained by nosespace analysis. Two major compounds were followed and tentatively identified at m/z 47.0494, (C₂H₆O)H⁺ corresponding mainly to ethanol and m/z 137.1325 corresponding mainly to limonene. These identifications were confirmed by an off-line measurement using static headspace GC-MS.

### In vivo aroma release

Assessor exhaled air was sampled via two glass tubes inserted into the nostril and fixed on laboratory glasses [1]. This tailor-made nosepiece allowed the subject to breathe comfortably during eating or drinking. The majority of the breath-air was released into the room. Only 80ml/min was drawn into the PTR-TOF-MS (Ionicon, Austria) via its transfer line connected to the nosepiece. To avoid condensation, the transfer line was heated at 100°C. A 1/8 inch copper tubing of 20cm length was inserted inside the PTR-TOF-MS transfer line and around its 1/16 inch inlet capillary peek tubing passing the heated transfer line. Due to the high copper thermal conductivity it was possible to heat the capillary peek tubing until its extremity.

The PTR-TOF-MS was set-up to monitor a full spectrum from m/z 10 to 350 every 0.1s. Internal mass scale calibration was done on a parasitic ion always present, m/z 29.9974(NO)⁺ and acetone coming from usual air lab contamination and, as body metabolite, also present in breath air at m/z 59.0491 (C₃H₆O)H⁺.

Assessor breathing pattern were also followed on m/z 59.0491 (C₃H₆O)H⁺ corresponding to acetone.

### In vivo oral processing

UltraSound imaging was acquired using a Siemens SC2000 (Siemens, Renens, Switzerland) used in parallel to observe in real time the drinking process by maintaining the ultrasonic probe under the oral cavity.

To synchronize precisely the acquisition time of the PTR-TOF-MS and the UltraSound instrument, a 5mV trigger (amplified to 1.6V for the PTR-TOF-MS) was recorded on analog input of both instruments (analogue input in PTR-TOF-MS and ElectroCardioGraphy input for the Ultrasound).

### Results

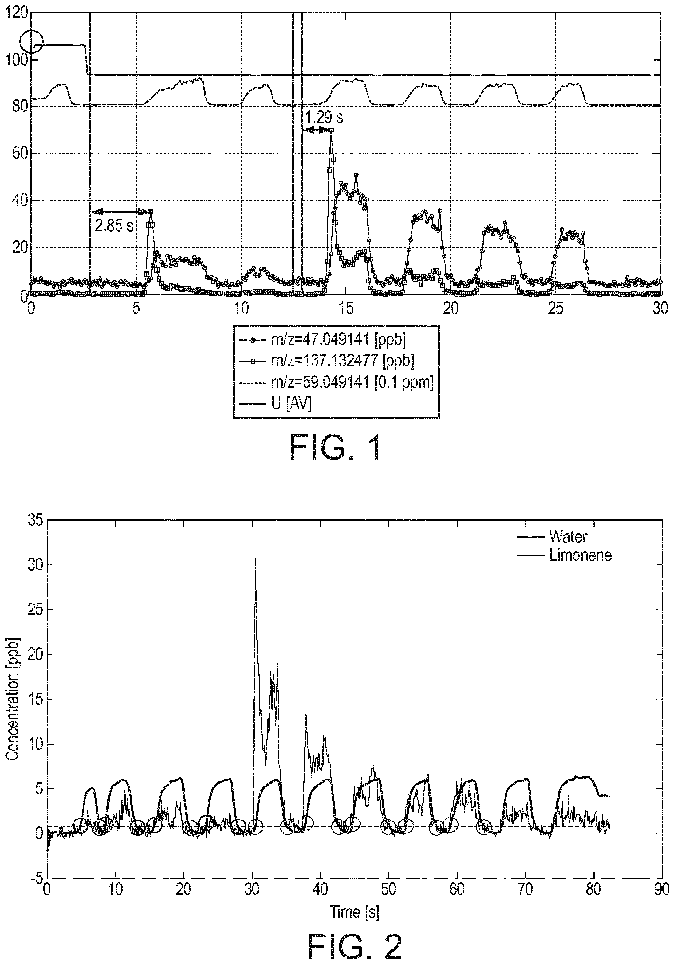

Using this setup, the inventors were able to determine the presence of volatile compounds in the subject's breath. In Figure 1, one can see that three events (first contact in mouth, swallowing start, swallowing end, all materialized by thick vertical lines) were identified using the ultrasound images. First the product inlet in the mouth was recorded around 2.5s after the beginning of the experiment as normalized using the two equipments. The first peak of limonene was then measured 2.85s after the first contact of the product in mouth. Limonene (m/z 137.1325) quickly rarefies in the nose space prior to swallowing, whilst ethanol (m/z 47.0491) sustains its intensity over two breath cycles prior swallowing. The initiation of swallowing and completion of swallowing are very close to one another (Δt=0.2s) and result in no aroma released during the swallowing phase itself. 1.29s after the completion of the swallowing phase, a new burst of limonene can be measured, which again quickly rarefies whilst ethanol rarefaction in the nose space is much slower. This period of 1.29s of delay between the two signals gives us a first approximation of the time spent by the volatiles in the pharynx before exhalation.

### Example 2 - Detection of volatile compound in products with different viscosities

The method described in Example 1 was used with the following modifications:
1. PTR-QMS instrument was used instead of PTR-TOF-MS and the compounds were followed by their unit mass instead of exact mass.
2. Multiple ions detection mode was used to follow four ion traces every 0.1s; M/z 21, m/z 37 and compounds of interest m/z 47 (ethanol) and m/z 81 (limonene).

M/z 81 corresponding to a fragment of limonene was chosen instead of the protonated molecular ion (m/z 137) due to a better sensitivity. Assessor breathing pattern were followed on m/z 37 (water cluster) corresponding to the humidity in exhaled breath and m/z 21 as the primary ion was used to normalized the data.

The inventors analysed the detection of volatile compound in breath following the administration of products across a range of viscosities.

Five samples of orange syrup with different levels of thickener were tested (water, molasses 65g and 75g/100ml and Resource® ThickenUp Clear (TUC) 0.6 and 1.8%). Two repetitions were performed for each measurement and the slope of the decay of the area of the limonene peak was used to differentiate samples. The more negative the slope the faster the clearance from the oropharyngeal cavity.

In a high viscosity fluid - e.g. 65g/100ml molasses (M65) - the peaks after swallowing decayed slowly and the ratio the area of the first peak to the area of the four successive peaks [P1/(P2+P3+P4+P5)] was low, and there was a weakly negative slope (see Figure 2). This indicates that the high viscosity fluid is associated with the formation of residues due to a thicker boundary layer deposited on the pharynx, leading to slow clearance of the product.

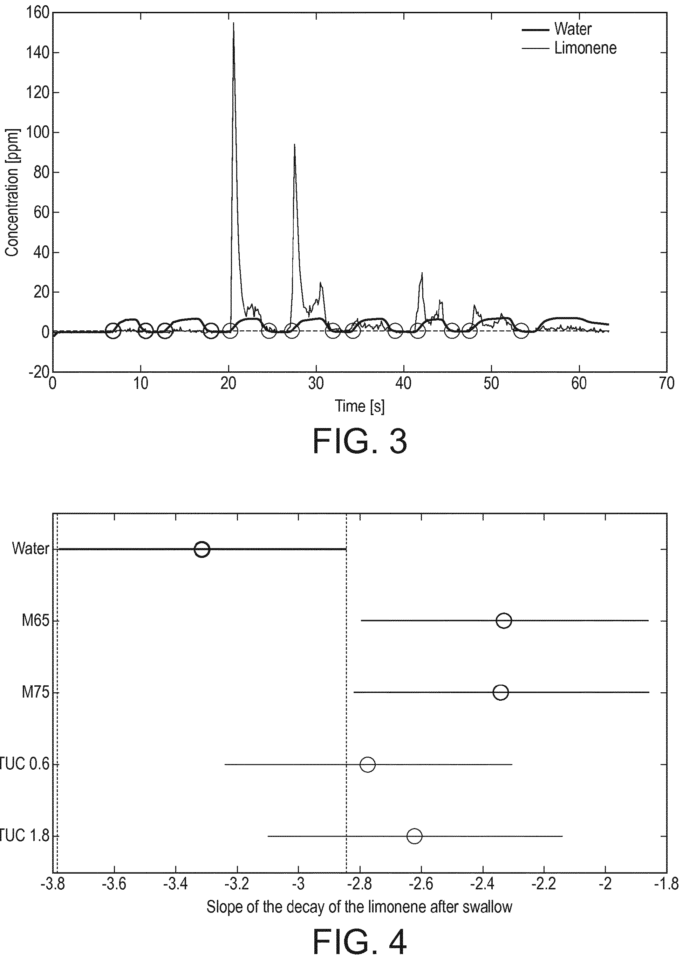

In a low viscosity fluid - e.g. water - the peaks after swallowing decayed quickly and the ratio the area of the first peak to the area of the four successive peaks [P1/(P2+P3+P4+P5)] was higher than for M65 and M75, and there was a strongly negative slope (see Figure 3). This indicates that the low viscosity fluid clears rapidly from the pharynx in healthy subjects.

A 2-way ANOVA (MATLAB 2015b, *anova2,* with the subjects as *random* factor) was used to assess the differences between the slopes yielded by the different products. The limonene signal detected from water decayed significantly faster (p<0.06) than in M65 (molasses 65g/100ml) and M75 (molasses 75g/100ml). Interestingly, TUC data are in between the water and molasses - suggesting it leaves fewer residues than the molasses thickened product (see Figure 4, data are also summarised in Table 2).

**Table 2**

| | Water | M65 | M75 | TUC06 | TUC18 |
|---|---|---|---|---|---|
| Molasse | | 65.00 g | 75.00 g | | |
| Resource® Thicken Up Clear (TUC) | | | | 0.60 g | 1.80 g |
| Orange syrup | 20.00 g | 20.00 g | 20.00 g | 20.00 g | 20.00 g |
| Vittel water / *Completed in a 100 ml graduated flask* | 100.00 ml | 100.00 ml | 100.00 ml | 100.00 ml | 100.00 ml |
| Slope | -3.35 | -2.4* | -2.4* | -2.8 | -2.6 |

| | | | | | |
|---|---|---|---|---|---|
| *significant difference (p<0.06) vs water (-3.35) | | | | | |

[1] Santo Ali, Philippe Pollien, Christian Lindinger and Chahan Yeretzian, in vivo analysis of aroma release while eating food: a novel set-up for monitoring on-line nosespace air, 1st International Conference on Proton Transfer Reaction Mass Spectrometry and Its Applications, 161-164, (2003). Although the present invention has been described in connection with specific preferred embodiments, it should be understood that the invention as claimed should not be unduly limited to such specific embodiments. Indeed, various modifications of the described modes for carrying out the invention, which are obvious to those skilled in the relevant fields, are intended to be within the scope of the following claims.

## Claims

1. A method for selecting a food product for a subject having dysphagia comprising:
(a) administering a food product comprising a volatile compound to the subject;
(b) detecting release of the volatile compound in exhaled breath of the subject during and/or after swallowing of the food product using mass spectrometry, a breath analyser / breathanalyser or a microfluidics chip;
(c) comparing the release of the volatile compound to a control;
(d) repeating steps (a) to (c) for at least one further food product comprising a volatile compound; and
(e) selecting the food product for which the release of the volatile compound in exhaled breath of the subject is most similar to the control.

2. A method according to claim 1 wherein the control is the release of the volatile compound
when the food product comprising the volatile compound is administered to a subject that does not have any condition which causes difficulty swallowing.

3. A method according to any preceding claim further comprising administering the selected food product to the subject.

4. A method according to any preceding claim wherein the food product comprising the volatile compound has been spiked or sprayed with the volatile compound.

5. A method according to any preceding claim wherein the food product is selected from a liquid or semi-solid food product.

6. A method according to any preceding claim wherein the food product has syrup or nectar consistency, custard or honey consistency, or pudding consistency.

7. A method according to any preceding claim wherein the food product is selected from water, milk, soup, yogurt, juice, coffee, tea, soda, or combinations thereof.

8. A method according to claim 7 wherein the volatile compound is detected by a method selected from a group consisting of proton transfer reaction mass spectrometry (PTR-MS), atmospheric-pressure chemical ionization mass spectrometry (APCI-MS), gas chromatography mass spectrometry (GC-MS) and gas chromatography ion-mobility mass spectrometry (GC-IMS).

9. A method according to any preceding claim wherein detecting release of the volatile compound after swallowing comprises detecting the levels of the volatile compound in exhaled breath over a period of time after swallowing.

10. A method according to claim 9 wherein detecting the release of the volatile compound in exhaled breath during and/or after swallowing of the food product is used to determine the extent of aspiration of the food product by the subject and/or the amount of residues of the food product in the oropharyngeal cavity of the subject.

11. A method according to any preceding claim wherein the volatile compound is selected from a group consisting of ethanol, limonene and ethyl butyrate, preferably ethanol.

12. A method according to any preceding claim which further comprises monitoring phases of the swallowing process in the subject, preferably wherein the swallowing process is monitored by ultrasound imaging and/or ultrasound Doppler velocimetry.

## Patentansprüche

1. Verfahren zum Auswählen eines Nahrungsmittelproduktes für ein Subjekt mit Dysphagie, umfassend:
(a) Verabreichen eines Nahrungsmittelproduktes, umfassend eine flüchtige Verbindung, an das Subjekt;
(b) Nachweisen der Freisetzung der flüchtigen Verbindung in ausgeatmetem Atem des Subjekts während und/oder nach dem Schlucken des Nahrungsmittelproduktes unter Verwendung von Massenspektrometrie, einem Atem-Analysator/Atemanalysator oder einem Mikrofluidik-Chip;
(c) Vergleichen der Freisetzung der flüchtigen Verbindung mit einer Kontrolle;
(d) Wiederholen der Schritte (a) bis (c) für mindestens ein weiteres Nahrungsmittelprodukt, umfassend eine flüchtige Verbindung; und
(e) Auswählen des Nahrungsmittelproduktes, für das die Freisetzung der flüchtigen Verbindung in ausgeatmetem Atem des Subjekts der Kontrolle am ähnlichsten ist.

2. Verfahren nach Anspruch 1, wobei die Kontrolle die Freisetzung der flüchtigen Verbindung ist, wenn das Nahrungsmittelprodukt, umfassend die flüchtige Verbindung, einem Subjekt verabreicht wird, das keinen Zustand aufweist, der Schwierigkeit beim Schlucken verursacht.

3. Verfahren nach einem der vorstehenden Ansprüche, ferner umfassend das Verabreichen des ausgewählten Nahrungsmittelproduktes an das Subjekt.

4. Verfahren nach einem der vorstehenden Ansprüche, wobei das Nahrungsmittelprodukt, umfassend die flüchtige Verbindung, mit der flüchtigen Verbindung versetzt oder besprüht wurde.

5. Verfahren nach einem der vorstehenden Ansprüche, wobei das Nahrungsmittelprodukt aus einem flüssigen oder halbfesten Nahrungsmittelprodukt ausgewählt ist.

6. Verfahren nach einem der vorstehenden Ansprüche, wobei das Nahrungsmittelprodukt Sirup- oder Nektarkonsistenz, Creme- oder Honigkonsistenz oder Puddingkonsistenz aufweist.

7. Verfahren nach einem der vorstehenden Ansprüche, wobei das Nahrungsmittelprodukt ausgewählt ist aus Wasser, Milch, Suppe, Joghurt, Saft, Kaffee, Tee, Soda oder Kombinationen davon.

8. Verfahren nach Anspruch 7, wobei die flüchtige Verbindung mittels eines Verfahrens nachgewiesen wird, ausgewählt aus einer Gruppe, bestehend aus Protonentransferreaktionsmassenspektrometrie (PTR-MS), chemischer lonisierungsmassenspektrometrie bei Atmosphärendruck (APCI-MS), Gaschromatographie-Massenspektrometrie (GC-MS) und Gaschromatographielonenmobilitätsmassenspektrometrie (GC-IMS).

9. Verfahren nach einem der vorstehenden Ansprüche, wobei das Nachweisen der Freisetzung der flüchtigen Verbindung nach dem Schlucken das Nachweisen der Spiegel der flüchtigen Verbindung in ausgeatmetem Atem über einen Zeitraum nach dem Schlucken umfasst.

10. Verfahren nach Anspruch 9, wobei das Nachweisen der Freisetzung der flüchtigen Verbindung in ausgeatmetem Atem während und/oder nach dem Schlucken des Nahrungsmittelproduktes verwendet wird, um das Ausmaß der Aspiration des Nahrungsmittelproduktes durch das Subjekt und/oder die Menge an Resten des Nahrungsmittelproduktes in dem oropharyngealen Hohlraum des Subjekts zu bestimmen.

11. Verfahren nach einem der vorstehenden Ansprüche, wobei die flüchtige Verbindung ausgewählt ist aus einer Gruppe, bestehend aus Ethanol, Limonen und Ethylbutyrat, vorzugsweise Ethanol.

12. Verfahren nach einem der vorstehenden Ansprüche, das ferner das Überwachen von Phasen des Schluckprozesses in dem Subjekt umfasst, vorzugsweise wobei der Schluckprozess mittels Ultraschallbildgebung und/oder Ultraschall-Doppler-Geschwindigkeitsmessung überwacht wird.

## Revendications

1. Procédé pour la sélection d'un produit alimentaire pour un sujet ayant une dysphagie comprenant :
(a) l'administration d'un produit alimentaire comprenant un composé volatil au sujet ;
(b) la détection de libération du composé volatil dans l'air expiré par le sujet pendant et/ou après déglutition du produit alimentaire en utilisant une spectrométrie de masse, un analyseur de gaz respiratoire/analyseur d'haleine ou une puce microfluidique ;
(c) la comparaison de la libération du composé volatil à un témoin ;
(d) la répétition des étapes (a) à (c) pour au moins un autre produit alimentaire comprenant un composé volatil ; et
(e) la sélection du produit alimentaire pour lequel la libération du composé volatil dans l'air expiré par le sujet est la plus similaire au témoin.

2. Procédé selon la revendication 1 dans lequel le témoin est la libération du composé volatil lorsque le produit alimentaire comprenant le composé volatil est administré à un sujet qui n'a pas d'état quelconque qui provoque une difficulté à déglutir.

3. Procédé selon une quelconque revendication précédente comprenant en outre l'administration du produit alimentaire choisi au sujet.

4. Procédé selon une quelconque revendication précédente dans lequel le produit alimentaire comprenant le composé volatil a été enrichi ou pulvérisé avec le composé volatil.

5. Procédé selon une quelconque revendication précédente dans lequel le produit alimentaire est choisi parmi un produit alimentaire liquide ou semi-solide.

6. Procédé selon une quelconque revendication précédente dans lequel le produit alimentaire a une consistance de sirop ou de nectar, une consistance de crème ou de miel, ou une consistance de pudding.

7. Procédé selon une quelconque revendication précédente dans lequel le produit alimentaire est choisi parmi de l'eau, du lait, de la soupe, du yaourt, du jus, du café, du thé, une boisson gazeuse ou des combinaisons de ceux-ci.

8. Procédé selon la revendication 7 dans lequel le composé volatil est détecté par un procédé choisi dans un groupe constitué par une spectrométrie de masse couplée à une réaction de transfert de protons (PTR-MS), une spectrométrie de masse couplée à une ionisation chimique à pression atmosphérique (APCI-MS), une spectrométrie de masse couplée à une chromatographie en phase gazeuse (GC-MS) et une spectrométrie de masse de mobilité ionique couplée à une chromatographie en phase gazeuse (GC-IMS).

9. Procédé selon une quelconque revendication précédente dans lequel la détection de la libération du composé volatil après déglutition comprend la détection des taux du composé volatil dans l'air expiré sur une période de temps après déglutition.

10. Procédé selon la revendication 9 dans lequel la détection de la libération du composé volatil dans l'air expiré pendant et/ou après la déglutition du produit alimentaire est utilisée pour déterminer le degré d'aspiration du produit alimentaire par le sujet et/ou la quantité de résidus du produit alimentaire dans la cavité oropharyngée du sujet.

11. Procédé selon une quelconque revendication précédente dans lequel le composé volatil est choisi dans un groupe constitué d'éthanol, limonène et butyrate d'éthyle, de préférence éthanol.

12. Procédé selon une quelconque revendication précédente qui comprend en outre la surveillance de phases du processus de déglutition chez le sujet, de préférence dans lequel le processus de déglutition est surveillé par imagerie ultrasonore et/ou vélocimétrie Doppler ultrasonore.
